# EUROPÄISCHE PATENTSCHRIFT

(11) **EP 1 842 504 B1**
(45) Veröffentlichungstag und Bekanntmachung des Hinweises auf die Patenterteilung: **19.11.2008**
(21) Anmeldenummer: 07006816.8
(22) Anmeldetag: 19.05.2003
(51) Int. Cl.: A61B 17/70

(54) **Verankerungselement zur Befestigung eines Stabes an einem Wirbelknochen**
Anchor element for attaching the rod of a device for treating a human or animal spine to a vertebra
Elément dýancrage destiné à la fixation d'une barre d'un dispositif pour régler une colonne vertébrale humaine ou animale sur une vertèbre

(30) Priorität: 21.05.2002 DE 20207852 U; 21.05.2002 DE 20207850 U
(43) Veröffentlichungstag der Anmeldung: 10.10.2007
(62) Teilanmeldung aus: 03727236.6
(73) Patentinhaber: Metz-Stavenhagen, Peter, 34537 Bad Wildungen (DE)
(72) Erfinder: Metz-Stavenhagen, Peter, 34537 Bad Wildungen (DE)
(74) Vertreter: Walther, Walther & Hinz GbR

(56) Entgegenhaltungen:
- EP-A- 1 190 678
- EP-A2- 0 885 598
- US-B1- 6 251 112
- US-B1- 6 258 090

## Beschreibung

Die vorliegende Erfindung betrifft ein Verankerungselement zur Befestigung eines Stabes einer Vorrichtung zum Einrichten.einer menschlichen oder tierischen Wirbelsäule an einem Wirbelknochen, mit einer im wesentlichen Uförmig ausgebildeten und zwei im wesentlichen parallel angeordnete Haltestege umfassende Halterung, in dem ein Aufnahmeschlitz zur Aufnahme des Stabes ausgebildet ist, und mit einem gegen den im Aufnahmeschlitz aufgenommenen Stab wirkenden Sicherungselement.

Aus dem US 6,258,090 B1 (Basis für den Oberbegriff des Anspruch 1) ist eine Pedikelschraube bekannt, die mit ihrem Gewindeschaft in einem Wirbelkörper verankerbar ist und die an ihrem aus dem Wirbelknochen herausragenden Ende eine U-förmig gebildete Halterung mit zwei parallel ausgerichteten Haltestegen aufweist. Dabei ist zwischen den Haltestegen ein Aufnahmeschlitz zur Aufnahme eines Distraktions- oder Kompressionsstabes ausgebildet, der mittels einem an den freien Enden der Haltestege befestigbaren Sicherungselement gesichert und fixiert werden kann. Dieses Sicherungselement umfasst ein erstes Schraubenelement, an dessen Außenseite zwei Viertelkreisgewinde ausgebildet sind und dessen innenseite mit einem Innengewinde ausgestattet ist. In dieses Innengewinde kann dann eine Madenschraube zur entgültigen Fixierung des Distraktions- öder Kompressionsstabes eingeführt werden. Zum Einsetzen des Schraubenelementes ist ein sehr aufwändiges Werkzeug notwendig, insbesondere da die Viertelkreisgewinde genauso groß ausgebildet sind, wie die zwischen den Viertelkreisgewinden verbleibenden Freiräume.

Davon ausgehend liegt der vorliegenden Erfindung die Aufgabe zugrunde, ein Verankerungselement zu schaffen, bei dem das Sicherungselement die auftretenden Kräfte zuverlässig aufnimmt und bei dem das Sicherungselement einfach und schnell anzubringen ist.

Als technische Lösung dieser Aufgabe wird erfindungsgemäß ein Verankerungselement mit den Merkmalen des Anspruches 1 vorgeschlagen. Vorteilhafte Weiterbildungen sind den Unteransprüchen zu entnehmen.

Ein nach dieser technischen Lehre ausgebildetes Verankerungselement hat den Vorteil, dass das Ringelement lediglich axial auf die freien Enden der Haltestege aufgesetzt werden braucht und durch ein leichtes Verschwenken in seine endgültige, gesicherte Position gebracht werden kann. Ein derart gesichertes Ringelement hält den im Aufnahmeschlitz befindlichen Stab bereits provisorisch in der gewünschten Position, so dass der operierende Arzt nun in Ruhe das Feststellelement einschrauben kann, um den Stab zu justieren und zu fixieren.

Ein weiterer Vorteil besteht darin, dass das Sicherungselement mit seinem Ringelement lediglich derart ausgerichtet werden braucht, dass seine Einführabschnitte den Haltestegen gegenüberliegen, und dass das Sicherungselement anschließend axial in die Halterung eingeführt werden braucht, um anschließend mit beispielsweise einer viertel Drehung die Gewindeabschnitte des Ringelementes mit den Teilgewinden der Haltestegen in Eingriff zu bringen. Schließlich wird das Feststellelement angezogen, um den Stab zu fixieren. Somit kann das Sicherungselement schnell und in einfacher Weise in die Halterung eingesetzt und dort fixiert werden.

Dabei sind die Gewindeabschnitte und die Teilgewinde als Sägezahngewinde ausgebildet, damit aufgrund der wirkenden Kräfte die Haltestege nicht auseinandergedrückt werden.

Die abgeflachte Stirnseite der Gewindestege hat den Vorteil, das der Gewindesteg hierdurch leicht in das Teilgewinde eingeführt werden kann. Dabei hat es sich als vorteilhaft erwiesen, dass derart ausgebildete Stirnseiten der Gewindestege zunächst einmal nur grob in das Teilgewinde eingeführt werden brauchen, um sich dann selbstständig in die korrekte Position zu ziehen.

Um ein einfaches, axiales Einführen des Sicherungselementes in die Halterung zu ermöglichen, hat es sich als vorteilhaft erwiesen, jeden Gewindeabschnitt über ein Kreissegment von vorzugsweise 60°, maximal jedoch auf ein Kreissegment von knapp 90° zu erstrecken. Hierdurch ist der über den Umfang gesehene freie Teil des Ringelementes größer, als der mit einem Gewindeabschnitt versehene Teil, so dass das Ausrichten des Ringelementes beim axialen Einführen in die Halterung nicht sehr präzise ausgeführt werden braucht.

In einer anderen, bevorzugten Ausführungsform sind die Gewindestege des Gewindeabschnittes an zumindest einer Stirnseite angefast oder abgerundet ausgebildet, damit der Gewindesteg leicht in das Teilgewinde eingeführt werden kann. Dabei hat es sich als vorteilhaft erwiesen, dass derart ausgebildete Stirnseiten der Gewindestege zunächst einmal nur grob in das Teilgewinde eingeführt werden brauchen, um sich dann selbstständig in die korrekte Position zu ziehen.

Weitere Vorteile des erfindungsgemäßen Verankerungselementes ergeben sich aus der beigefügten Zeichnung und den nachfolgend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Auftählung zu verstehen, sondern haben vielmehr beispielhaften Charakter. Es zeigen:
- Fig. 1: eine Explosionsdarstellung eines erfindungsgemäßen Verankerungselementes mit einem Stab;
- Fig. 2: eine Draufsicht auf die Halterung des Verankerungselementes gemäß Fig. 1;
- Fig. 3: eine Draufsicht auf das Sicherungselement des Verankerungsetementes gemäß Fig. 1;
- Fig. 4: eine perspektivische Ansicht des Verankerungselementes gemäß Fig. 1, wobei das Sicherungselement explosionsartig herausgezogen ist;
- Fig. 5: eine perspektivische Ansicht des Verankerungselementes gemäß Fig. 6, wobei das Sicherungselement in die Halterung eingesetzt ist;
- Fig. 6: eine Ausschnittsvergrößerung des Verankerungselementes gemäß Fig. 2;
- Fig. 7: eine Explosionsdarstellung einer zweiten Ausführungsform eines erfindungsgemäßen Verankerungselementes mit einem Stab;
- Fig. 8: eine Draufsicht auf die Halterung des Verankerungselementes gemäß Fig. 7;
- Fig. 9: eine Draufsicht auf das Sicherungselement des Verankerungselementes gemäß Fig. 7.

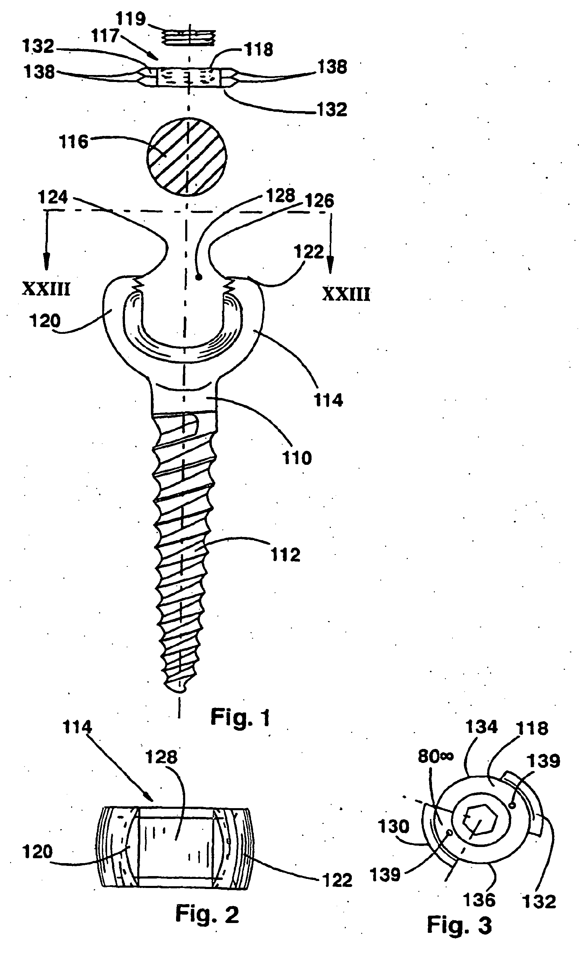

In den Fig. 1 -3 ist eine erste Ausführungsform eines erfindungsgemäßen Verankerungselomentes explasionsartig dargestellt. Dieses schraubenähnliche Verankerungselement 110 umfasst einen in den Wirbelknochen einschraubbaren Gewindeschaft, an dem eine Halterung 114 zur Befestigung eines Stabes 116 angebracht ist. Zum Verankerungselement 110 gehört auch ein Sicherungselement 117, welches ein Ringelement 118 und ein als Madenschraube ausgebildetes Feststellelement 119 umfasst. Das Sicherungselement 117 ist in die Halterung 114 einsetzbar und fixiert den Stab 116 in der Halterung 114.

Die Halterung 114 ist im Querschnitt U-förmig gebogen ausgeführt und umfasst zwei im Wesentlichen parallel angeordnete Haltestege 120, 122. Auf den sich jeweils zugewandten Seiten der Haltestege 120, 122 ist je ein Teilge winde 124, 126 ausgebildet, welches als Sägezahngewinde ausgeführt ist.

Unter einem Sägezahngewinde sind neben dem metrischen Sägengewinde gemäß DIN 513 auch Sägezahngewinde mit einem etwas größeren oder etwas kleineren Flankenwinkel, mit einem Flankenwinkel von 0° oder einem negative Flankenwinkel, sowie Sägezahngewinde gemäß EP 885 598 zu verstehen.

In der Hafterung 114 ist zwischen den Haltestegen 120.122 ein Aufnahmeschlitz 128 zur Aufnahme des Stabes 116 ausgebildet. Zur Fixierung dieses Stabes wird das Sicherungselement 117 mit seinem Ringelement 118 in die Teilgewinde 124, 126 eingesetzt und drückt somit den Stab 116 tief in den Aufnahmeschlitz 128 hinein und fixiert desselben.

Damit das Ringelement 118 zuverlässig in die Teilgewinde 124, 126 der Haltestege 120, 122 eingreifen kann, befinde sich auf dem Umfang des Ringelementes 118 zwei Gewindeabschnitte 130, 132. Wie in der Draufsicht gemäß Fig. 3 zu erkennen ist, liegen sich die Gewindeabschnitte 130,132 genau gegenüber, während zwischen den jeweiligen Gewindeabschnitten 130, 132 Einführabschnitte 134, 136 ausgebildet sind. Das Im Querschnitt kreisrunde Ringelement 118 hat außerhalb der Gewindeabschnitte 130, 132 einen Durchmesseer von maximal dem Kemdurchmesser der Gewindeabschnitte 130, 132. Im Bereich der Gewindeabschnitte 130, 132 sind auf das Ringelement 118 jeweils zwei Gewindestege 138 aufgebracht, die den jeweiligen Gewindeabschnitt 130, 132 bilden. Analog zu den Teilgewinden 124, 126 sind auch die Gewindeabschnitte 130,132 als Sägezahngewinde ausgeführt. Die Gewindeabschnitte 130, 132 erstrecken sich über ein Kreissegment von 80 und sind exakt gegenüberliegend angeordnet. Auf einer Oberseite des Ringelementes 118 sind zwei Werkzeugaufnahmen 139 vorgesehen, in die ein entsprechendes Werkzeug einsetzbar ist, mit dem das Ringelement 118 in die Halterung 114 einschraubbar ist. Im Ringelement 118 ist über ein Gewinde ein als Madenschraube ausgebildetes Feststellelement 119 gehalten, welches unabhängig vom Ringelement 118 einschraubbar ist, beispielsweise um den Stab endgültig zu fixieren. Dieses Feststellelement 119 besitzt eine Sechskantaufnahme 140, so dass das Feststellelement 119 mit einem Sechskant beliebig fest angezogen werden kann.

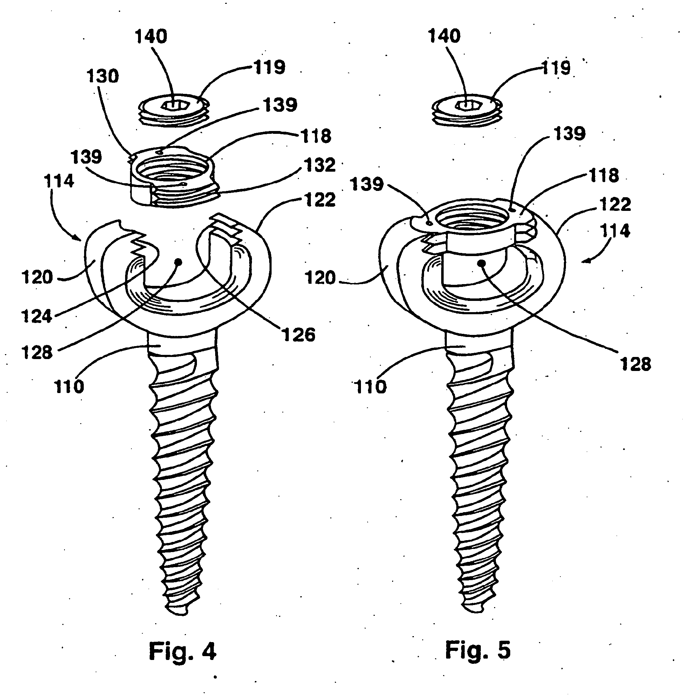

In den Fig. 4 und 5 ist das Verankerungselement 110 perspektivisch in zwei verschiedenen Positionen dargestellt, wobei Fig. 4 das Sicherungselement 117 oberhalb des eigentlichen Verankerungselementes 110 zeigt, während das Sicherungselement 117 in Fig. 5 in die Halterung 114 eingesetzt dargestellt ist. Zum Einsetzen des Sicherungselementes 117 wird das Ringelement 118 mit einer hier nicht dargestellten Zange oder einem ähnlichen Werkzeug ergriffen und anschließend axial in den Aufnahmeschlitz 128 der Aufnahme 114 eingeführt. Dabei ist das Ringelement 118 so ausgerichtet, dass dessen Einführäbschnitte 134, 136 den Teilgewinden 124, 126 der Haltestegen 120, 122 gegenüberliegen, während die Gewindeabschnitte 130, 132 in Richtung des Aufnahmeschlitzes 128 ausgerichtet sind und in dieser Position über die Halterung 114 hinausragen. Ist das Ringelement 118 tief genug in den Aufnahmeschlitz 128 eingeführt, so wird es um 90 ° verschwenkt. Nun werden die Gewindeabschnitte 130, 132 in das jeweilige Teilgewinde 124, 12 hineingeführt. In diesem Zustand fixiert das Ringelement 118 den Stab in der Halterung 114, wobei das Sägezahngewinde ein Auseinanderdrücken der Haltestege 120, 122 verhindert und somit eine zuverlässige Befestigung des Stabes im Verankerungselement 110 gewährleistet.

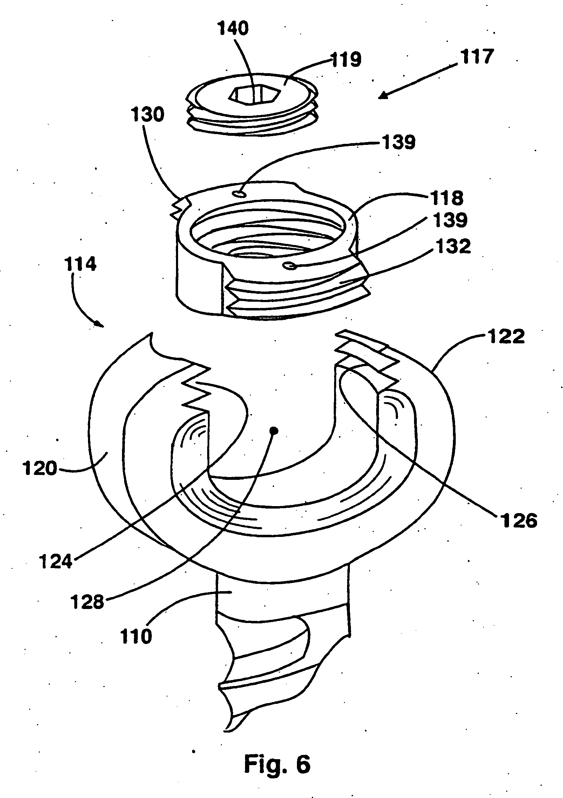

In der in Fig. 6 dargestellten Detailvergrößerung ist gut zu erkennen, dass die Gewindestege 138 der Gewindeabschnitte 130, 132 an zumindest einer Stirnseite abgeflacht ausgebildet sind. Hierdurch wird das Einführen der Gewindeabschnitte 130, 132 in die Teilgewinde 124, 126 erleichtert, da die Gewindeabschnitte 130, 132 lediglich ungefähr in das Teilgewinde 124, 126 eingesetzt werden brauchen und sich ansonsten selbst in die richtige Position bewegten.

Durch das Reduzieren des Gewindes des Ringelementes 118 auf zwei sich gegenüberliegende Fast-Viertel-Gewinde ist es möglich, das Sicherungselement 117 im gewindefreien Bereich schnell und einfach an den Teilgewinden 124, 126 der Haltestege 120, 122 vorbeizuführen und durch eine 90°-Drehung in diese einzuführen. Dies erleichtert die Arbeit des operierenden Chirurgen deutlich und führt zu einer signifikanten Verkürzung der Operationsdauer.

Der Stab 116 kann beispielsweise als Kompressions-, Verbindungs-oder Distraktionsstab ausgebildet sein, je nach Einsatzzweck.

In einer anderen, hier nicht dargestellten Ausführungsform ist ein Teilgewinde 124 und/oder ein Teilgewinde 126 im Anschlag vorgesehen, so dass das Ringelement 118 nicht versehentlich zu weit gedreht wird und wieder aus der Halterung 114 herausfällt.

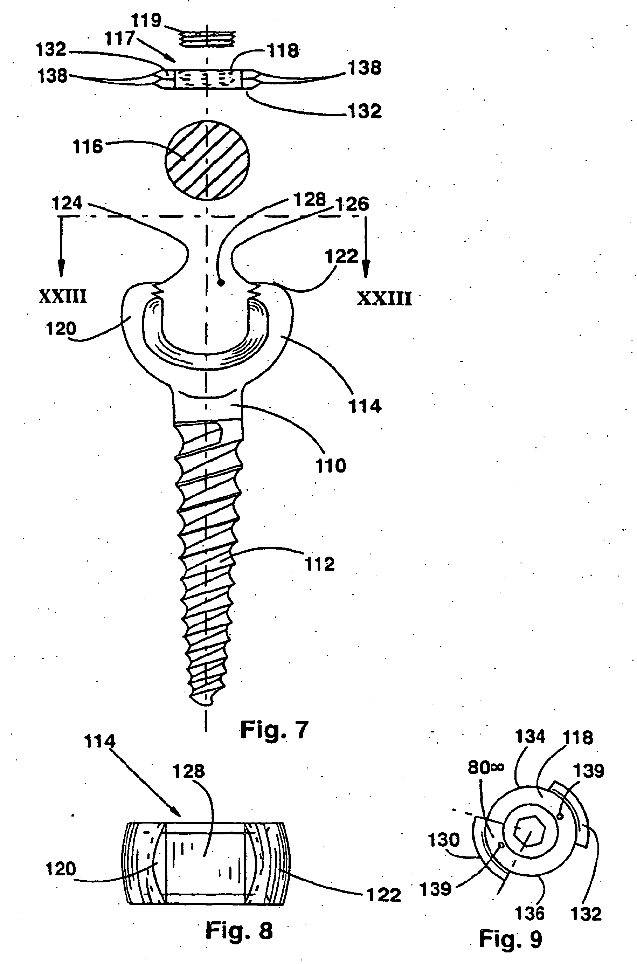

In den Figuren 7 bis 9 ist noch eine weitere Ausführungsform eines erfindungsgemäßen Verankerungselementes 150 dargestellt, welches sich durch die Ausgestaltung der Halterung 152 und eines dementsprechend angepassten Ringelements 154 von der ersten Ausführungsform unterscheidet. Im Unterschied zur ersten Ausführungsform sind die Haltestege 156, 158 der Halterung 152 breiter dimensioniert, als die in den Haltestegen 156,158 angebrachten Teilgewinde 160, 162, so dass die Teilgewinde 160, 162 nicht bis an den jeweiligen Rand der Haltestege.156, 158 reichen, sondern von diese beabstandet endes.

Wie aus Figur 9 besonders gut hervorgeht, ist das Ringelement 154 an seinen Einführabschnitten 164 und 166 mit einer vergleichsweise glatten und konvex gewölbten Oberfläche ausgebildet, um in einfacher Weise in den Aufnahmeschlitz 128 zwischen den Haltestegen 156,158 eingeführt und dort verdreht zu werden, bis die Gewindeabschnitte 168,170 in die jeweiligen Teilgewinde 160, 162 eingreifen, und das Ringelement 154 zuverlässig halten.

### Bezugszeichenliste:

- 110: Verankerungselement
- 112: Gewindeschaft
- 114: Halterung
- 116: Distraktions-oder Kompressionsstab
- 117: Sicherungselement
- 118: Ringelement
- 119: Feststellelement
- 120: Haltesteg
- 122: Haltesteg
- 124: Teilgewinde
- 126: Teilgewinde
- 128: Aufnahmeschlitz
- 130: Gewindeabschnitt
- 132: Gewindeabschnitt
- 134: Einführabschnitt
- 136: Einführabschnitt
- 138: Gewindesteg
- 139: Werkzeugaufnahme
- 140: Sechskantaufnahme
- 150: Verankerungselement
- 152: Halterung
- 154: Ringelement
- 156: Haltestege
- 158: Haltestege
- 160: Teilgewinde
- 162: Teilgewinde
- 164: Einführungsabschnitt
- 166: Einführungsabschnitt
- 168: Gewindeabschnitt
- 170: Gewindeabschnitt

## Patentansprüche

1. Verankerungselement zur Befestigung eines Stabes einer Vorrichtung zum Einrichten einer menschlichen oder tierischen Wirbelsäule an einem Wirbelkörper, mit einer im wesentlichen U-förmig ausgebildeten und zwei im wesentlichen parallel angeordnete Haltestege (120,122) umfassenden Halterung (114), in der ein Aufnahmeschlitz (128) zur Aufnahme des Stabes (116) ausgebildet ist und mit einem gegen den im Aufnahmeschlitz (128) aufgenommen Stab (116) wirkenden Sicherungselement (117), wobei die Haltestege (120, 122) an sich gegenüberliegenden Seiten ein Teilgewinde (124, 126) aufweisen, wobei das Sicherungselement (117) ein Feststellelement (119) und ein Ringelement (118) umfasst, wobei am Umfang des Ringelementes (118) zwei sich gegenüberliegende Gewindeabschnitte (130, 132) und zwei sich gegenüberliegende Einführabschnitte (134, 136) ausgebildet sind, wobei der Durchmesser des Ringelementes (118) im Bereich der Einführabschnitte (134, 136) nicht größer als der Kerndurchmesser des Gewindeabschnittes (130,132) ist, so dass die Gewindeabschnitte (130, 132) mit den Teilgewinden (124, 126) der Haltestege (120, 122) in Eingriff bringbar sind,
**dadurch gekennzeichnet,**
**dass** die Gewindeabschnitte (130, 132) und die Teilgewinde (124, 126) ein Sägezahngewinde aufweisen, und dass ein Gewindesteg (138) des Gewindeabschnittes (130, 132) an einer Stirnseite abgeflacht augebildet ist.

2. Verankerungselement nach Anspruch 1,
**dadurch gekennzeichnet,**
**dass** sich der Gewindeabschnitt (130, 132) über ein Kreissegment von schätzungsweise 80 °, maximal 90° erstreckt.

3. Verankerungselement nach einem der vorangehenden Ansprüche,
**dadurch gekennzeihnet,**
dass ein Gewindesteg (138) des Gewindeabschnittes (130, 132) an einer Stirnseite angefast oder abgerundet ausgebildet ist.

## Claims

1. An anchoring element for fastening a rod of an apparatus for setting a human or an animal spine to a vertebral bone, with a substantially U-shaped holding device (114) comprising two substantially parallel holding ridges (120, 122) in which there is formed a receiving slot (128) for receiving the rod (116) and with a securing element (117) acting against the rod (116) received in the receiving slot (128), said holding ridges (120, 122) comprising a partial thread (124, 126) on opposing sides, said securing element (117) including a fixing element (119) and a ring element (118), two opposing threaded portions (130, 132) and two opposing insertion portions (134, 136) being formed on the circumference of the ring element (118), the diameter of the ring element (118) in the region of the insertion portions (134, 136) not being greater than the core diameter of the threaded portion (130, 132) for the threaded portions (130, 132) to be movable into engagement with the partial thread (124, 126) of the holding ridges (120, 122),
**characterized in**
**that** the threaded portions (130, 132) and the partial threads (124, 126) comprise a buttress thread and that a flattened thread ridge (138) of the threaded portion (130, 132) is formed on one end side.

2. The anchoring element as set forth in claim 1,
**characterized in**
**that** the threaded portion (130, 132) extends over a segment of a circle of approximately 80°, of 90° maximum.

3. The anchoring element as set forth in any one of the previous claims,
**characterized in**
**that** a chamfered or rounded thread ridge (138) of the threaded portion (130, 132) is formed on one end side.

## Revendications

1. Elément d'ancrage destiné à la fixation d'une tige d'un dispositif destiné à réduire une colonne vertébrale humaine ou animale sur un corps vertébral, avec un dispositif de retenue (114) conformé sensiblement en U et comprenant deux branches de retenue (120, 122) sensiblement parallèles dans lequel est ménagée une fente (128) de réception d'une tige (116) et avec un élément de sécurisation (117) agissant à l'encontre de la tige (116) reçue dans la fente (128) de réception, les branches de retenue (120, 122) comportant sur des faces situées en regard une partie de filetage (124, 126), l'élément de sécurisation (117) comprenant un élément de fixation (119) et un élément annulaire (118), deux segments filetés (130, 132) situés en regard et deux segments d'introduction (134, 136) situés en regard étant formés sur la circonférence de l'élément annulaire (118), le diamètre de l'élément annulaire (118) dans la zone des segments d'introduction (134, 136) n'étant pas supérieur au diamètre du noyau du segment fileté (130, 132) de sorte que les segments filetés (130, 132) sont aptes à se mettre en prise avec les parties de filetage (124, 126) des branches (120, 122),
**caractérisé en ce**
**que** les segments filetés (130, 132) et les parties de filetage (124, 126) comportent un filetage en dents de scie et qu'un filet (138) du segment fileté (130, 132) présente une forme aplatie sur une face d'extrémité.

2. Elément d'ancrage selon la revendication 1,
**caractérisé en ce**
**que** le segment fileté (130, 132) s'étend sur une portion de cercle d'environ 80°, sur 90° au plus.

3. Elément d'ancrage selon l'une quelconque des revendications précédentes,
**caractérisé en ce**
**qu'**un filet (138) du segment fileté (130, 132) présente une forme biseautée ou arrondie sur une face d'extrémité.
